# EUROPEAN PATENT SPECIFICATION

(11) **EP 2 877 484 B1**
(45) Date of publication and mention of the grant of the patent: **11.10.2017**
(21) Application number: 13823892.8
(22) Date of filing: 26.07.2013
(51) Int. Cl.: C07K 14/765

(54) **A METHOD FOR POLISHING ALBUMIN**
VERFAHREN ZUM POLIEREN VON ALBUMIN
PROCÉDÉ DE POLISSAGE D'ALBUMINE

(30) Priority: 27.07.2012 US 201261676799 P; 23.08.2012 EP 12181515; 14.03.2013 US 201313826500; 11.04.2013 AU 2013203968
(43) Date of publication of application: 03.06.2015
(73) Proprietor: CSL Limited, Parkville VIC 3052 (AU)
(72) Inventor: McCANN, Karl Beaumont, Hoppers Crossing Victoria 3029 (AU); BERTOLINI, Joseph, Ashburton Victoria 3147 (AU)
(74) Representative: Binsack, Beate
(86) International application number: PCT/AU2013/000836
(87) International publication number: WO 2014/015388

(56) References cited:
- US-A1- 2006 234 907
- HONG-FEI TONG ET AL: "Enhancing IgG purification from serum albumin containing feedstock with hydrophobic charge-induction chromatography", JOURNAL OF CHROMATOGRAPHY A, vol. 1244, 1 June 2012 (2012-06-01), pages 116-122, XP055048519, ISSN: 0021-9673, DOI: 10.1016/j.chroma.2012.04.073
- ARAKAWA T ET AL: "MEP HyperCel chromatography II: Binding, washing and elution", PROTEIN EXPRESSION AND PURIFICATION, ACADEMIC PRESS, SAN DIEGO, CA, vol. 71, no. 2, 1 June 2010 (2010-06-01), pages 168-173, XP026969372, ISSN: 1046-5928, DOI: 10.1016/J.PEP.2009.11.004 [retrieved on 2009-11-12]
- ZHAO G ET AL: "5-Aminoindole, a new ligand for hydrophobic charge induction chromatography", JOURNAL OF CHROMATOGRAPHY, ELSEVIER SCIENCE PUBLISHERS B.V, NL, vol. 1211, no. 1-2, 21 November 2008 (2008-11-21), pages 90-98, XP025589329, ISSN: 0021-9673, DOI: 10.1016/J.CHROMA.2008.09.108 [retrieved on 2008-10-29]
- TANAKA, K. ET AL.: 'Purification of human albumin by the combination of the method of Cohn with liquid chromatography' BRAZILIAN JOURNAL OF MEDICAL AND BIOLOGICAL RESEARCH vol. 31, no. 11, November 1998, pages 1383 - 1388, XP055175157
- TONG, H.-F. ET AL.: 'Enhancing IgG purification from serum albumin containing feedstock with hydrophobic charge-induction chromatography' JOURNAL OF CHROMATOGRAPHY A vol. 1244, June 2012, pages 116 - 122, XP055048519

## Description

### TECHNICAL FIELD

The present invention relates generally to a method of purifying albumin from solution. More specifically, to a method of polishing an albumin enriched solution through a hydrophobic charge-induction chromatographic resin.

### BACKGROUND

The majority of commercial human serum albumin (HSA) is manufactured using ethanol precipitation methods based on those originally developed by Cohn *et al.* [1946] and the subsequent modifications of Kistler & Nitschmann [1962]. The separation using these methods is based on the solubility of plasma proteins at varying ethanol concentrations, pH, ionic strength and temperature. However, a major disadvantage of the Cohn purification process for the purification of albumin is the use of ethanol concentrations up to 40% v/v and the requirement of the procedures to be performed at temperatures less than 0°C. Nevertheless, the sustained success of albumin purification by ethanol precipitation is largely attributed to the fact that these methods are very efficient at processing the large volumes encountered in a modern plasma fractionation process.

Smaller but increasing amounts of albumin are now being fractionated using chromatographic processes, either as a final polishing step following initial ethanol fractionation [More & Harvey, 1991 or a fully integrated chromatographic process [Marrs, 1993; Yap *et al.,* 1993; Martins *et al.,* 2011; Berglof *et al.,* 1983]. Chromatographic purification of HSA has a number of benefits including higher yields, higher purity [More & Harvey, 1991; Yap *et al.,* 1993; Véron *et al.,* 1993], lower aggregates and improved tolerability and clinical safety [Che *et al.,* 2006]. The chromatography method originally developed by Berglof *et al.* [1983], for example, was initially applied to a Supernatant II + III feedstream and includes the use of two ion exchange and size exclusion columns. This method was later adapted to a delipidated Supernatant I feedstream to accommodate the high IgG yielding chromatographic process for Intragam P [Yap *et al.,* 1993; Bertolini *et al.*, 1999].

Despite these benefits, the widespread adoption of chromatographic processes for fractionation of human albumin has been limited due to inefficiencies associated with operation of these processes on an industrial scale. In addition, the anion and cation exchange chromatography steps used in methods such as those reported by Berglof *et al.* [1983] involve the capture/binding of albumin to the resin. The positive binding of albumin to the anion and cation exchange columns means that the protein loadings are restricted to ≤ 100 g/L. This has limited industrial application given that human plasma contains albumin as the predominant protein (approx. 60 -70% of total protein) and the chromatographic purification of albumin would therefore require large columns (≥ 200 L) and multiple cycles. This is particularly evident for the fully chromatographic processes, which employ a gel filtration step for final polishing of HSA to remove high molecular weight impurity proteins. The low flow rates and small sample loads required to maintain effective separation make this step a significant bottleneck in the chromatographic albumin manufacturing process.

The present invention solves, or at least partly alleviates, the problems in the art by providing a more efficient method for polishing an albumin enriched solution.

### SUMMARY OF THE INVENTION

In an aspect of the present invention, there is provided a method of polishing albumin to remove contaminants comprising passing an albumin enriched solution through a hydrophobic charge-induction chromatographic resin and recovering the albumin solution which passes through the resin.

In an embodiment, the hydrophobic charge-induction chromatographic resin comprises 4-mercaptoethylpyridine.

In an embodiment, the albumin enriched solution is selected from Cohn Supernatant I, Cohn Supernatant II+III, Supernatant or Filtrate from Kistler-Nitschmann, Cohn Supernatant IV and Cohn Fraction V. In an embodiment, the albumin enriched solution is a diafiltered Cohn Supernatant I. In an embodiment, the Supernatant or Filtrate from Kistler-Nitschmann Nitschmann is Supernatant/Filtrate A or B.

In an embodiment, the albumin enriched solution is a flow through solution recovered when plasma or diafiltered Cohn Supernatant I is passed through an expanded bed adsorption resin in negative mode with respect to albumin.

In an embodiment, the albumin enriched solution is delipidated and euglobulin depleted.

In an embodiment, prior to passing the albumin enriched solution through the hydrophobic charge-induction chromatographic resin, the method comprises the steps of (a) passing the solution through an anion-exchange resin in negative mode with respect to albumin and (b) passing the solution through a cation exchange resin in negative mode with respect to albumin. In an embodiment, the method comprises passing the flow through fraction from the anion-exchange resin of step (a) through the cation-exchange resin in step (b). In another embodiment, the method comprises passing the flow through fraction from the cation-exchange resin of step (b) through the anion-exchange resin in step (a).

In an embodiment, the method comprises pasteurizing the polished albumin solution.

Also disclosed herein is a polished albumin solution recovered by the methods disclosed herein.

Also disclosed herein is a pasteurized albumin solution produced by the methods disclosed herein.

Furthermore described herein is a composition comprising a polished albumin solution and having a turbidity of less than 5 NTU. In an example, the composition comprises:
(a) greater than 80 mM sodium;
(b) greater than 19% w/v albumin;
(c) less than 4 µg/mL IgA;
(d) less than 0.1 mg/mL IgG;
(e) less than 0.03 mg/mL Apolipoprotein A1;
(f) less than 0.07 mg/mL α1-proteinase inhibitor; and/or
(g) less than 1% w/v aggregates/polymers.

Furthermore described herein is a polished albumin solution comprising at least 19% w/v total protein and any one or more of the following:
(a) an albumin content of at least 95% of total protein;
(b) less than 2% w/v aggregate, preferably less than 1% w/v aggregate;
(c) a dimer content of less than 2% w/v, more preferably less than 1.5% w/v;
(d) a monomer content of at least 97% w/v;
(e) a fragment content of less than 0.5% w/v;
(f) less than 28.6 IU/mL of prekallikrein activator (PKA), more preferably less than 10 IU/mL PKA, even more preferably less than 1 IU/mL PKA;
(g) less than 0.1% w/v α₂-macroglobulin;
(h) less than 0.01% w/v Apolipoprotein A1;
(i) less than 0.05% w/v transferrin;
(j) less than 0.05% w/v α₁-glycoprotein;
(k) less than 0.1% w/v α₁-proteinase inhibitor, preferably less than 0.05% w/v, α₁-proteinase inhibitor;
(l) less than 0.05% w/v haptoglobin;
(m) less than 0.1% w/v inter-α-trypsin inhibitor;
(n) from about 45 mmol/L to about 100 mmol/L sodium, preferably from about 80 mmol/L to 90 mmol/;
(o) less than 5 µg/mL IgA, preferably less than 2 µg/mL IgA;
(p) less than 0.2 mg/mL IgG, preferably less than 0.01 mg/mL IgG;
(q) less than 110 µg/L aluminium, less than 80 µg/L aluminium, more preferably less than 30 µg/L aluminium;
(r) less than 40 mmol/L caprylate;
(s) osmolality of about 130 mosmol/kg to about 140 mosmol/kg; and
(t) turbidity of less than 6 NTU, preferably less than 3 NTU.

### BRIEF DESCRIPTION OF THE FIGURES

**Figure 1** shows the impurity break through profile for α2-macroglobulin during MEP Hypercel purification of pH adjusted CM eluate. The results show the amount of impurity (α2-macroglobulin) detected in the flow-through, explored over a sample volume of up to 1000 mL.
**Figure 2** shows the impurity break through profile for IgG during MEP Hypercel purification of pH adjusted CM eluate. The results show the amount of impurity (IgG) detected in the flow-through, explored over a sample volume of up to 1000 mL.
**Figure 3** shows the impurity break through profile for IgA during MEP Hypercel purification of pH adjusted CM eluate. The results show the amount of impurity (IgA) detected in the flow-through, explored over a sample volume of up to 1000 mL.
**Figure 4** shows the impurity break through profile for IgM during MEP Hypercel purification of pH adjusted CM eluate. The results show the amount of impurity (IgM) detected in the flow-through, explored over a sample volume of up to 1000 mL.

### DETAILED DESCRIPTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

It must be noted that, as used in the subject specification, the singular forms "a", "an" and "the" include plural aspects unless the context clearly dictates otherwise. Thus, for example, reference to "a resin" includes a single resin, as well as two or more resins; reference to "the composition" includes a single composition, as well as two or more compositions; and so forth.

In the absence of any indication to the contrary, reference made to a "%" content throughout this specification is to be taken as meaning % w/v (weight/volume).

Albumin and IgG are two of the most commercially important products extracted from plasma. By the Cohn process, under specific conditions of ethanol concentrations, temperature, conductivity and pH, an intermediate fraction (Supernatant I) is generated that is enriched in IgG and albumin. Fraction II + III and Supernatant II + III are then generated, which are enriched in IgG and albumin, respectively. Further purification is then achieved by manipulating the above parameters. However, a major disadvantage of the Cohn purification process is the use of ethanol and the requirement of the procedures to be performed at temperatures less than 0°C. The purification of albumin in particular requires high concentrations of ethanol (up to 40% w/v for Cohn Fractions IV and V).

The present invention is predicated, in part, on the finding that passing an albumin enriched solution through a hydrophobic charge-induction chromatographic resin in negative mode with respect to albumin and recovering the albumin solution which passes through the resin is an efficient alternative to existing chromatographic processes for polishing (purifying) albumin in solution, the resulting polished albumin solution having greater purity than is available using existing chromatographic processes. It has also been found that, by combining an ion exchange chromatographic step prior to polishing through a hydrophobic charge-induction chromatographic resin, the method of the present invention can provide an albumin solution with an even higher level of purity than existing processes. When combined with the fact that these chromatography steps can be incorporated into existing downstream industrial processes for the purification of albumin, it offers the plasma fractionation industry an efficient separation technique that complements the other fractionation steps.

In an aspect of the present invention, there is provided a method of polishing albumin to remove high molecular weight contaminants comprising passing an albumin enriched solution through a hydrophobic charge-induction chromatographic resin and recovering the albumin solution which passes through the resin.

Methods of fractionating blood-derived products, such as albumin, from impurities (*e.g*., high molecular weight impurities such as immunoglobulin) using chromatography would be familiar to persons skilled the art. Most chromatographic processes employ a solid support, also referred to interchangeably herein as a resin or matrix. Suitable solid supports would be familiar to persons skilled in the art and the choice will invariably depend on the type of product to be purified. Examples of suitable solid supports include inorganic carriers, such as glass and silica gel, organic, synthetic or naturally occurring carriers, such as agarose, cellulose, dextran, polyamide, polyacrylamides, vinyl copolymers of bifunctional acrylates, and various hydroxylated monomers, and the like. Commercially available carriers are sold under the names of Sephadex™, Sepharose™, Hypercel™, Capto™, Fractogel™, MacroPrep™, Unosphere™, GigaCap™, Trisacryl™, Ultrogel™, Dynospheres™, Macrosorb™ and XAD™ resins.

The chromatography steps will generally be carried out under non-denaturing conditions and at convenient temperatures in the range of about -10°C to +30°C, more usually at about ambient temperatures. The chromatographic steps may be performed batch-wise or continuously, as convenient. Any convenient method of separation may be employed, such as centrifugation, filtration, decanting, or the like.

Hydrophobic Charge Induction Chromatography (HCIC) will be familiar to persons skilled in the art. HCIC uses binding moieties attached to a solid support, wherein the binding moieties have specificity for the analyte that, in accordance with the methods of the present invention, are high molecular weight impurities (*e.g*., immunoglobulin). HCIC differs from other types of affinity chromatography, such as Hydrophobic Interaction Chromatography (HIC), which offers selectivity to separate plasma proteins based on differences in the surface hydrophobicity of the proteins [Goheen & Matson, 1985; Ramos-Clamont *et al.,* 2006]. The selectivity afforded by HIC has enabled it to be used successfully for the purification of albumin from human plasma [Rucheton *et al.,* 1997], human placenta [Cabrera-Crespo *et al.,* 2000] and for recombinant HSA derived from *Pichia pastoris* [Ohmura *et al.,* 1995]. However, despite HIC offering an orthogonal mode of separation to the ion exchange steps used for chromatographic purification of albumin, these matrices or resins are not used by any of the commercial plasma fractionators. A contributing factor that limits adoption of HIC at large-scale is the fact that these methods require use of significant amounts of lyotropic salts in order to achieve the appropriate separation conditions [Burton & Harding, 1998]. Some applications require use of these salts at high concentrations for the equilibration buffer and the sample in order to achieve the desired separation. Furthermore, the presence of high concentrations of salt in the albumin fraction requires extensive dialysis/diafiltration before the various viral inactivation and final formulation steps.

Any suitable HCIC resin known to persons skilled in the art can be used. In an embodiment, the hydrophobic charge-induction chromatographic resin comprises 4-mercaptoethylpyridine (MEP) (*e.g.,* MEP Hypercel). The MEP ligand has an advantage in that it permits separation based on the surface hydrophobicity of proteins, but does not require the addition of lyotropic salts often seen in other processes for the purification of albumin [Burton & Harding, 1998; Schwartz *et al.,* 2001]. MEP Hypercel has been shown to have a high selectivity for immunoglobulins and, for this reason, there has been a major focus on the use of this media for the isolation of monoclonal antibodies [Schwartz *et al.,* 2001; Ghose *et al.,* 2006; Arakawa *et al.,* 2010; Pezzini *et al.,* 2011]. In addition, MEP Hypercel has proven to be effective for the separation of penicillin acylase [Coulon *et al.,* 2004] and Fc-fusion proteins [Arakawa *et al.,* 2009].

In contrast to traditional hydrophobic interaction chromatography, HCIC is controlled on the basis of pH, rather than salt concentration.

MEP Hypercel resin is composed of cellulose matrix to which 4-MercaptoEthyl- Pyridine (4-MEP) is linked. The cellulose bead confers high porosity, chemical stability, and low non-specific interaction. The bead size of MEP Hypercel is about 80-100 µm to allow a good compromise between capacity and flow property. MEP also displays high selectivity and capacity for high molecular weight immunoglobulin impurities, and has a pKa of about 4.8. It contains a hydrophobic tail and an ionizable headgroup. At physiological pH, the aromatic pyridine ring is uncharged and hydrophobic. Additional contributions to binding are provided by the aliphatic spacer arm. Binding to immunoglobulin, for example, is further enhanced by interaction with the other group. Both ligand structure and ligand density are designed to provide effective binding in the absence of lyotropic or other salts. When pH of the mobile phase is adjusted to values below 4.8 (typically pH 4.0), the ligand takes on a distinct positive charge. Under such conditions, immunoglobulins also carry a positive charge. Electrostatic repulsion is induced and the immunoglobulins are desorbed. The pH-dependent behaviour of the ionisable MEP ligand confers a dual-mode property, which permits proteins to interact through hydrophobic interactions and elution to occur through electrostatic repulsion. Traditional hydrophobic interaction media are based on alkyl groups that are non-ionisable.

MEP Hypercel can therefore be used as a polishing step in the manufacture of albumin. The method can be applied to the polishing of albumin initially purified by ion exchange chromatography. Integrated laboratory-scale batches can therefore be manufactured using MEP Hypercel, with the resultant albumin having a greater purity than the existing process utilising resins, such as Sephacryl S200HR.

MEP Hypercel resin provides high binding capacity and high flow rates, ideal for small- and large-scale antibody purification applications.

In an embodiment, the albumin enriched solution is selected from Cohn Supernatant I, Cohn Supernatant II+III, Supernatant/Filtrate A, Cohn Supernatant IV and Fraction V. In an embodiment, the albumin enriched solution is a diafiltered Cohn Supernatant I.

Thus, in an embodiment, the albumin enriched solution is a flow through solution recovered when plasma or diafiltered Cohn Supernatant I is passed through an expanded bed adsorption resin in negative mode with respect to albumin.

In an embodiment, the albumin enriched solution is delipidated and euglobulin depleted.

The expression "albumin enriched solution", as used herein, is used interchangeably with expressions such as "crude albumin fraction" to denote a solution comprising albumin and high molecular weight impurities, such as immunoglobulins.

In an embodiment, prior to passing the albumin enriched solution through the hydrophobic charge-induction chromatographic resin, the method comprises the steps of (a) passing the solution through an anion exchange resin in negative mode with respect to albumin and (b) passing the solution through a cation exchange resin in negative mode with respect to albumin.

Ion exchange (anion or cation exchange) chromatography would be familiar to persons skilled in the art and represents one of the most common methods for the purification of proteins and other charged molecules from solution. In *cation* exchange chromatography, positively charged molecules are attracted to a negatively charged solid support. Conversely, in *anion* exchange chromatography, negatively charged molecules are attracted to a positively charged solid support.

A negatively charged solid support (as used in cation exchange chromatography) and a positively charged solid support (as used in anion exchange chromatography) can be prepared by any means known to persons skilled in the art and usually involves the covalent attachment of a charged functional group onto the solid support. Suitable charged functional groups would be familiar to persons skilled in the art and the type of functional group that is employed will invariably depend on the molecule to be separated from solution. Examples of suitable *cation* exchange resins are ones comprising a functional sulfonic group (S or SP) or a functional carboxymethyl group (CM). Examples of suitable anion exchange resins are ones comprising a functional quaternary amine group (Q) and/or a tertiary amine group (DEAE), or a diethylaminopropyl group (ANX). In an embodiment of the present invention, the anion-exchange matrix comprises diethylaminoethanol covalently linked to a polysaccharide polymer. In an embodiment of the present invention, the cation-exchange matrix comprises carboxymethyl groups covalently linked to a polysaccharide polymer.

In an embodiment of the present invention, the conditions for performing the ion exchange chromatography step will favour the removal of impurities from the albumin solution, such as immunoglobulins (*e.g*., IgG, IgA and IgM).

To optimize binding of charged impurities in the albumin enriched solution to the oppositely charged functional groups attached to the solid support, the albumin solution will generally be of low to medium conductivity (*i*.*e*., have a low to medium salt concentration). The adsorption of the charged impurities in the albumin solution to the solid support will be driven by the ionic interaction between the oppositely charged ionic groups in the impurities and the functional ligand on the support. The strength of the interaction is determined by the number and location of the charges on the impurities and on the functional group. By increasing the salt concentration (generally by using a linear salt gradient), the molecules with the weakest ionic interactions (*i*.*e*., albumin) will start to elute from the column first and be recovered in the flow through fraction. Molecules that have a stronger ionic interaction (*e.g*., impurities such as immunoglobulins) require a higher salt concentration and elute later in the gradient. The binding capacities of ion exchange resins are generally quite high. This is of major importance in industrial-scale chromatography, but is less critical for analytical-scale applications.

Persons skilled in the art will know that changes in pH can also affect separation by ion exchange chromatography. In cation exchange chromatography, for example, raising the pH of the mobile phase buffer will cause the molecule to become less protonated and hence less positively charged. The result is that the protein no longer can form an ionic interaction with the negatively charged solid support, which ultimately results in the molecule to elute from the column. In anion exchange chromatography, lowering the pH of the mobile phase buffer will cause the molecule to become more protonated and hence more positively (and less negatively) charged. The result is that the protein no longer can form an ionic interaction with the positively charged solid support that causes the molecule to elute from the column.

The pH of the mobile phase buffer can be between the pI (isoelectric point) or pKa (acid dissociation constant) of the charged molecule and the pKa of the charged group on the solid support. For example, in cation exchange chromatography, using a functional group on the solid support with a pKa of 1.2, a sample molecule with a pI of 8.2 may be run in a mobile phase buffer of pH 6.0. In anion exchange chromatography, a molecule with a pI of 6.8 may be run in a mobile phase buffer at pH 8.0 when the pKa of the solid support is 10.3.

A gradient of linearly increasing salt concentration can also be applied to elute the bound components (*i.e.,* impurities) from the solid support after the albumin enriched solution has been recovered, if necessary. An alternative to using a linear gradient is to use a step gradient. This requires less complicated equipment and can be very effective to elute different fractions if the appropriate concentrations of salt are known, usually from linear gradient experiments. The chromatography steps according to the present invention are performed in negative mode with respect to albumin, where impurities in the starting albumin solution are retained on the charged solid support and the albumin is recovered in the flow through fraction. The impurities (anions or cations) are retained on the stationary phase (*i*.*e*., solid support) and can be subsequently eluted, if necessary, by increasing the concentration of a similarly charged species that will displace the impurity ions from the stationary phase. For example, in cation exchange chromatography, the positively charged impurity could be displaced by the addition of positively charged sodium ions.

Chromatography can be performed using either axial flow columns, such as those available from GE Healthcare, Pall Corporation and Bio-Rad, or using radial flow columns, such as those available from Proxcys. Chromatography can also be conducted using expanded bed technologies.

The method of the present invention may also employ the use of multiple ion exchange chromatography steps to improve the purification of albumin. The use of multiple ion exchange chromatography steps may employ more than one anion exchange resin or more than one cation exchange resin. The method may also employ multiple anion and cation exchange resins (*i*.*e*., more than one anion exchange resin and more than one cation exchange resin).

Persons skilled in the art will understand that it does not matter in which order the multiple ion exchange chromatography steps of the present invention are performed. Thus, in one embodiment, the flow through fraction from the anion exchange resin of step (a) is passed through the cation exchange resin of step (b). In another embodiment, the flow through fraction from the cation exchange resin of step (b) is passed through an anion exchange resin of step (a).

In an embodiment, where the albumin enriched solution is passed through the ion exchange resins, the albumin enriched flow through fractions from steps (a) and/or (b) can be collected and stored indefinitely for subsequent fractionation (purification) in accordance with the methods of the present invention. In another embodiment, the albumin enriched flow through fraction recovered from steps (a) and (b) is passed directly through the hydrophobic charge induction resin in accordance with the methods of the present invention.

As the chromatography steps according to the present invention are to be performed in negative mode with respect to albumin, it would be understood by persons skilled in the art that the conditions for the ion exchange chromatographic steps according to the present invention will favour the retention of impurities to the solid support, allowing the albumin to the recovered in the flow through fraction. Given that the albumin purity in plasma is initially about 60-70%, chromatography steps that bind the lower abundant impurity proteins are significantly more efficient than those that capture albumin. Typical protein loads for albumin capture on ion exchange resins such as DEAE Sepharose-FF are 50 - 65 g/L and 50 - 100 g/L for albumin capture on ion exchange resins such as CM Sepharose-FF. The use of negative mode chromatography for the anion and cation exchange chromatography steps may permit loadings of ≥ 500 g/L.

In an embodiment of the present invention, the methods do not comprise an intervening chromatographic step in positive mode with respect to the albumin between step (a) and step (b). However, the methods of the present invention may comprise additional steps performed in positive mode with respect to albumin, where those additional steps are not performed between ion exchange chromatographic steps (a) or (b). For example, Capto DEAE resin can be used in positive mode (*i.e*., to capture albumin) in order to separate the crude IgG fraction in the flow through, then elute the albumin from the Capto DEAE resin for subsequent purification by the methods according to the present invention.

In another aspect of the present invention, there is provided a polished albumin solution recovered by the methods disclosed herein.

In an embodiment, the method further comprises pasteurizing the polished albumin solution. It has been found that the methods of the present invention result is a polished albumin solution that has less turbidity when pasteurized in comparison to the turbidity level (as measure by Nephelometric Turbidity Units; NTU) found in purified albumin solutions prepared by existing chromatographic processes. The level of turbidity is a measure of the presence of high molecular weight impurities in the albumin solution (*e.g.,* immunoglobulin). In an embodiment, the pasteurized albumin solution has a turbidity of less than 5 NTU.

Furthermore, there is provided a pasteurized albumin solution produced by the methods disclosed herein.

The composition or polished albumin solution provided herein comprises at least 4% w/v purified albumin. In some examples, the composition or polished albumin solution of the present invention comprises from 4% to 5% w/v purified albumin. In some examples, the composition or polished albumin solution of the present invention comprises greater than 19% w/v purified albumin. In some examples, the composition or polished albumin solution of the present invention comprises from 20% to 25% w/v purified albumin.

In another example, there is provided a composition comprising a pasteurized albumin solution and having a turbidity of less than 5 NTU. In a further example, the composition comprises:
(a) greater than 80mM sodium;
(b) greater than 19% w/v albumin;
(c) less than 4 µg/mL IgA;
(d) less than 0.1 mg/mL IgG;
(e) less than 0.03 mg/mL Apolipoprotein A1;
(f) less than 0.07 mg/mL α1-proteinase inhibitor; and/or
(g) less than 1% w/v aggregates/polymers

In another example, there is provided a polished albumin solution comprising at least 19% w/v total protein and any one or more of the following:
(a) an albumin content of at least 95% of total protein;
(b) less than 2% w/v aggregate, preferably less than 1% w/v aggregate;
(c) a dimer content of less than 2% w/v, more preferably less than 1.5% w/v;
(d) a monomer content of at least 97% w/v;
(e) a fragment content of less than 0.5% w/v;
(f) less than 28.6 IU/mL of prekallikrein activator (PKA), more preferably less than 10 IU/mL PKA, even more preferably less than 1 IU/mL PKA;
(g) less than 0.1 % w/v α₂-macroglobulin;
(h) less than 0.01% w/v Apolipoprotein A1;
(i) less than 0.05% w/v transferrin;
(j) less than 0.05% w/v α₁-glycoprotein;
(k) less than 0.1% w/v α₁-proteinase inhibitor, preferably less than 0.05% w/v, α₁-proteinase inhibitor;
(l) less than 0.05% w/v haptoglobin;
(m) less than 0.1% w/v inter-α-trypsin inhibitor;
(n) from about 45 mmol/L to about 100 mmol/L sodium, preferably from about 80 mmol/L to 90 mmol/;
(o) less than 5 µg/mL IgA, preferably less than 2 µg/mL IgA;
(p) less than 0.2 mg/mL IgG, preferably less than 0.01 mg/mL IgG;
(q) less than 110 µg/L aluminium, less than 80 µg/L aluminium, more preferably less than 30 µg/L aluminium;
(r) less than 40 mmol/L caprylate;
(s) osmolality of about 130 mosmol/kg to about 140 mosmol/kg; and
(t) turbidity of less than 6 NTU, preferably less than 3 NTU.

Those skilled in the art will appreciate that the invention described herein is susceptible to variations and modifications other than those specifically described. It is to be understood that the invention includes all such variations and modifications which fall within the spirit. The invention also includes all of the steps, features, referred to or indicated in this specification, individually or collectively, and any and all combinations of any two or more of said steps or features.

Certain embodiments of the invention will now be described with reference to the following examples which are intended for the purpose of illustration only and are not intended to limit the scope of the generality hereinbefore described.

### EXAMPLES

### Materials and Methods

***Protein solutions:*** Purified human serum albumin (HSA) and purified human immunoglobulin G (IgG) was obtained from CSL Biotherapies (Broadmeadows, Victoria, Australia). Process intermediates including Cohn Supernatant I (SNI) depleted of lipids and euglobulins, albumin eluate from CM Sepharose-FF chromatography and purified albumin from Sephacryl S200HR chromatography were obtained from CSL Biotherapies (Broadmeadows, Victoria, Australia). All protein solutions were filtered through a 0.22 µm membrane (Durapore^{®}, Millipore Corporation, Bedford, MA, USA) prior to chromatography.

*A**ntibodies and ELISA reagents:*** Antibodies against the human plasma proteins of transferrin, α₂-macroglobulin, α₁-glycoprotein, α₁-antitrypsin, inter α trypsin inhibitor, haptoglobin, apolipoprotein A1, apolipoprotein B and ceruloplasmin, which were used for immunoelectrophoresis, were obtained from The Binding Site (Birmingham, England), Siemens Healthcare Diagnostics, Inc. (New York, USA) and Dako Denmark A/S (Glostrup, Denmark). ELISA kits for IgG, IgM, AMG were obtained from Bethyl Laboratories Inc. (Texas, USA). Antibodies and IgA standard for the IgA ELISA were obtained from Dako Denmark (Glostrup, Denmark). Antibodies against the human plasma proteins of albumin, IgG, IgA and IgM, which were used for nephelometry analysis, were obtained from (Beckman-Coulter Inc. (Fullerton, CA, USA).

***Chromatographic** r**esins:*** MEP Hypercel resin was obtained from Pall Life Sciences (East Hills, NY, USA). DEAE Sepharose-FF, Capto DEAE, CM Sepharose-FF and Sephacryl S200HR resins were obtained from GE Healthcare BioSciences AB (Uppsala, Sweden).

### Example 1: Characterisation of Sephacryl S200HR process

Before commencing development studies for the MEP Hypercel, the current Sephacryl S200HR polishing step was fully characterised to identify the major contaminant proteins present in the albumin intermediate and understand the effectiveness of the Sephacryl S200HR at removing these contaminants. The albumin intermediates before and after Sephacryl S200HR chromatography were characterised using nephelometry (IMMAGE Immunochemistry system, Beckman-Coulter Inc., Fullerton, CA, USA) to determine the levels of albumin, IgG, IgA and IgM, and immunoelectrophoresis to determine the levels of transferrin, α₂-macroglobulin, α₁-glycoprotein, α₁-antitrypsin, inter α trypsin inhibitor, haptoglobin, apolipoprotein A1, apolipoprotein B and ceruloplasmin.

The concentrated ion exchange albumin intermediate which is loaded on to the Sephacryl S200HR contained 1.42% impurity proteins, of which α₂- macroglobulin, IgG and IgA were the predominant proteins (Table 1). These major impurity proteins all have molecular weights significantly higher than that of albumin, thus making the Sephacryl S200HR gel filtration media well suited to the removal of these contaminants. The Sephacryl S200HR chromatography step was effective at removing the high molecular weight impurities, with clearance rates of greater than 94% observed for α₂-macroglobulin, IgA and IgM, and 74.0% clearance of IgG. Following Sephacryl S200HR chromatography the total impurity content was reduced to 0.19%, with IgG being the most abundant impurity protein present.

**Table 1: Characterisation of albumin solution before and after Sephacryl S200HR chromatography (NT = Not Tested)**

| | **Albumin before Sephacryl S200HR** | | **Albumin after Sephacryl S200HR** | | **Clearance across S200HR** |
|---|---|---|---|---|---|
| **Component** | **Concentration (mg/mL)** | **% of total** | **Concentration (mg/mL)** | **% of total** | **%** |
| Albumin | 146.0 | - | 155.1 | - | |
| IgG | 0.544 | 0.37 | 0.150 | 0.096 | 74.0 |
| IgA | 0.374 | 0.26 | 0.012 | 0.008 | 97.0 |
| IgM | 0.110 | 0.08 | 0.007 | 0.004 | 94.0 |
| Transferrin | <0.021 | - | NT | - | - |
| α₂-macroglobulin | 0.911 | 0.62 | 0.026 | 0.017 | 97.3 |
| α₁-glycoprotein | <0.011 | - | NT | - | - |
| α₁-antitrypsin | 0.073 | 0.05 | 0.045 | 0.029 | 42.0 |
| Inter-α-trypsin inhibitor | <0.125 | - | NT | - | - |
| Haptoglobin | 0.035 | 0.02 | NT | - | - |
| Apolipoprotein A1 | 0.034 | 0.02 | 0.053 | 0.034 | -46.7 |
| Apolipoprotein B | <0.057 | - | NT | - | - |
| Ceruloplasmin | <0.018 | - | NT | - | - |
| **Total** | | 1.42% | | 0.19% | |

### Example 2 : Impact of heating on turbidity of albumin solutions

The safety record of commercial albumin solutions is attributed largely to the pasteurisation step (60°C for 10 hours) [More & Harvey, 1991]. The albumin solution is stabilised by addition of sodium caprylate, however the presence of contaminant proteins which are not protected by the caprylate are susceptible to heat induced denaturation during pasteurisation. A control sample of albumin intermediate eluated from CM Sepharose-FF chromatography and intermediate samples of purified albumin derived from Sephacryl S200HR chromatography were concentrated to 15-20% w/v protein by ultrafiltration (10 kDa OMEGA membrane; Pall Life Sciences, USA) and formulated with 32 mM sodium caprylate. These samples were not subjected to the low pH caprylate incubation step. The samples were then pasteurised by heating at 60°C for 10 hours. At the completion of pasteurisation, the turbidity of the samples was assessed using a Hach 2100AN Turbidimeter (Hach Company, Loveland, CO, USA).

Despite the albumin eluate derived from CM Sepharose-FF chromatography having a purity of over 98%, and thus complying with the albumin Pharmacopoeial requirements for protein composition (and purity), the removal of high molecular weight impurities is critical for the quality of the final albumin product. This is clearly demonstrated by the fact that when the albumin derived from CM Sepharose-FF chromatography is concentrated, formulated with sodium caprylate and subjected to pasteurisation at 60°C for 10 hours a significant increase in turbidity is observed (Table 2). No turbidity increase is encountered when the purified albumin derived from Sephacryl S200HR is pasteurised.

**Table 2: Effect of pasteurisation on turbidity of albumin derived from ion exchange chromatography and Sephacryl S200 chromatography**

| | **Turbidity (NTU)** | |
|---|---|---|
| **Sample** | **Before pasteurisation** | **After pasteurisation** |
| Sephacryl S200 eluate | 2.23 | 2.36 |
| CM Sepharose-FF eluate | 2.75 | 9.36 |

### Example 3 : Determination of MEP Hypercel operating conditions

The operating parameters for MEP Hypercel chromatography were explored using purified samples of albumin and IgG. The binding and elution of albumin and IgG was determined separately using a range of equilibration conditions including; 50 mM sodium phosphate (pH 7.0); 50 mM sodium acetate (pH 5.5); 50 mM sodium acetate (pH 5.5) + 150 mM NaCl; 50 mM sodium acetate (pH 5.5) + 300 mM NaCl; 50 mM sodium acetate (pH 7.0); and 110 mM sodium acetate (pH 7.0). The purified IgG and purified albumin samples were prepared in the appropriate equilibration buffer at a concentration of approximately 5-10 mg/mL and filtered through a 0.22 µm membrane (Durapore^{®}, Millipore Corporation, Bedford, MA, USA) prior to chromatography. The samples were loaded on to an equilibrated MEP Hypercel column (17.5 cm x 1.6 cm ID; GE Healthcare BioSciences AB, Uppsala, Sweden) at 20 g per litre of resin. The unbound protein was recovered during the sample loading period and during the subsequent post-sample wash using equilibration buffer. The bound protein was eluted using sodium acetate buffer at pH 3.0. All chromatography steps were conducted at a flow rate of 100 cm/h and monitored at 280 nm using an AKTA Explorer 900 equipped with Unicorn software (GE Healthcare BioSciences AB, Uppsala, Sweden). The amount of protein recovered in the flow through and eluate fractions was determined using nephelometry.

The most suitable equilibration buffer condition from the abovementioned experiment was verified using a sample containing a 9:1 mixture of albumin and IgG. The purified albumin and purified IgG was mixed in 110 mM Sodium acetate (pH 7.0) at a ratio of 9:1 to give a final protein concentration of approximately 5 - 10 mg/mL. The sample was loaded on to the equilibrated MEP Hypercel column at protein loadings of 100, 200 and 300 g/L. The unbound protein was recovered using 110 mM Sodium acetate (pH 7.0) equilibration buffer and the bound protein was eluted using sodium acetate buffer at pH 3.0. The amount of albumin and IgG in the flow through and eluate fractions was determined using nephelometry.

As the albumin sample before and after Sephacryl S200HR chromatography contained IgG as one of the major impurity proteins, the initial development of the MEP Hypercel chromatography step focussed on its ability to separate albumin from IgG. The effectiveness of MEP Hypercel to purify albumin was initially assessed by monitoring the binding and elution characteristics of pure samples of human albumin and human IgG using sodium phosphate (pH 7.0) and sodium acetate (pH 5.5) equilibration buffers. Previous studies by Schwartz *et al.* [2001] showed that MEP Hypercel has a maximum binding capacity when the sample pH is adjusted to approximately pH 7.0. The separation of albumin and IgG using MEP Hypercel was also evaluated at pH 5.5 as the albumin is currently eluted from the CM Sepharose-FF column at pH 5.5 using a sodium acetate buffer.

The sodium phosphate buffer at pH 7.0 was effective at binding IgG, with undetectable levels of IgG being recovered in the flow through fraction (Table 3). Under the same conditions, only minor amounts of albumin bound to the MEP Hypercel, and greater than 85% of the albumin recovered in the flow through fraction. Equilibration of the MEP Hypercel with the sodium acetate buffer at pH 5.5 resulted in conditions where approximately half of both the IgG and albumin bound to the resin. The addition of sodium chloride to the sodium acetate buffer (pH 5.5) improved IgG binding, with undetectable IgG levels being recovered in the flow through fraction (Table 3). The addition of sodium chloride also improved albumin recovery in the drop through fraction. However despite this improvement, approximately 15-20% of the albumin was recovered in the eluate fraction, which was significantly higher than the recovery obtained when the MEP Hypercel was equilibrated using the sodium phosphate buffer at pH 7.0.

Given that sodium acetate buffers are currently used in the chromatographic albumin process during the ion exchange chromatography steps, the binding and elution characteristics of purified albumin and IgG on the MEP Hypercel resin was assessed using sodium acetate buffers at pH 7.0. Both the 50 mM and 110 mM sodium acetate buffers at pH 7.0 resulted in similar IgG and albumin recoveries to the 50 mM sodium phosphate buffer, with undetectable levels of IgG being detected in the flow through fraction and only minor amounts of albumin (approximately 5% of total) being detected in the eluate (Table 3). The results suggest that the pH of the equilibration buffer and sample may influence binding of the impurities (*e.g*., immunoglobulins) to the MEP Hypercel resin, whereas the buffer type and conductivity of the equilibration buffer and sample have very little impact on the separation when operating at the optimal pH.

In the current chromatographic albumin process, albumin is eluted from the CM Sepharose-FF column using a 110 mM sodium acetate buffer (pH 5.5). As the MEP Hypercel showed good selectivity between albumin and IgG when equilibrated using a 110 mM sodium acetate buffer at pH 7.0 this buffer was selected for further evaluation. By selecting these conditions, it means that the albumin eluted from the CM Sepharose column would only require a pH adjustment step prior to loading on to the MEP Hypercel column.

**Table 3: Percentage recovery of albumin and IgG in flow through and eluate fractions collected from MEP Hypercel chromatography using different equilibration buffers (ND = Not detectable)**

| **Buffer conditions** | **Albumin** | | **IgG** | |
|---|---|---|---|---|
| | **Flow through** | **Eluate** | **Flow through** | **Eluate** |
| 50 mM sodium phosphate (pH 7.0) | 86.9 | 5.3 | ND | 63.9 |
| 50 mM sodium acetate (pH 5.5) | 44.7 | 39.6 | 42.1 | 68.1 |
| 50 mM sodium acetate (pH 5.5) + 150 mM NaCl | 79.9 | 17.3 | ND | 97.3 |
| 50 mM sodium acetate (pH 5.5) + 300 mM NaCl | 81.1 | 14.6 | ND | 90.9 |
| 50 mM sodium acetate (pH 7.0) | 104.5 | 5.2 | ND | 96.0 |
| 110 mM sodium acetate (pH 7.0) | 89.2 | 5.1 | ND | 90.5 |

Using the optimal equilibration conditions determined from Table 3, the separation characteristics of MEP Hypercel was evaluated using a 9:1 mixture of albumin and IgG. The MEP Hypercel showed excellent separation of the albumin and IgG mixture. At protein loadings of up to 200 g/L, the flow through fraction contained undetectable levels of IgG and over 90% of the total albumin loaded (Table 4). Most of the IgG was recovered in the eluate fraction and only minor amounts of albumin were lost in this fraction. The results suggest that the MEP Hypercel can remove significant amounts of IgG from an albumin solution that has a similar composition to the ion exchange eluate in the chromatographic albumin process.

**Table 4: Percentage recovery of albumin and IgG in flow through and eluate fractions collected from MEP Hypercel chromatography at different protein loadings of a 9:1 albumin:IgG mixture (ND = Not detectable)**

| | **Albumin** | | **IgG** | |
|---|---|---|---|---|
| **Protein loading** | **Flow through** | **Eluate** | **Flow through** | **Eluate** |
| 100 g/L | 92.0 | 1.8 | ND | 92.1 |
| 200 g/L | 90.0 | 1.3 | ND | 86.9 |
| 300 g/L | 98.2 | 0.9 | 1.4 | 86.2 |

### Example 4 : Purification of ion exchange purified albumin using MEP Hypercel

After demonstrating that the MEP Hypercel could successfully separate an albumin and IgG mixture, the ability of MEP Hypercel to purify the ion exchange purified albumin was assessed. The albumin eluate from CM Sepharose-FF chromatography was adjusted to pH 7.0 ± 0.1 and clarified by passage through a 0.22 µm filter (Durapore^{®}, Millipore Corporation, Bedford, MA, USA) prior to loading on to the MEP Hypercel column. The albumin sample (1000 mL) was loaded on to the MEP Hypercel column (17.5 cm x 1.6 cm ID), which had been equilibrated with 110 mM Sodium acetate (pH 7.0), followed by a 110 mM Sodium acetate (pH 7.0) post-sample wash to recover unbound protein and bound protein was removed by washing with 100 mM Sodium acetate (pH 3.0) and 1 M NaOH. The flow through fraction was collected at 10 mL intervals and assessed for the levels of the major impurity proteins of IgG, IgA, IgM and α₂-macroglobulin by ELISA.

The MEP Hypercel was further evaluated by assessing its ability to purify the albumin eluate from the CM Sepharose-FF column. As shown previously in Table 1, the albumin prior to Sephacryl S200HR chromatography, which contains the same composition as the eluate from the CM Sepharose-FF column, contained other impurity proteins including α₂-macroglobulin, IgA and IgM in addition to IgG. These four impurity proteins were used as purity markers for the evaluation of MEP Hypercel to evaluate the selectivity of the MEP Hypercel to the major impurities present in the chromatographic albumin.

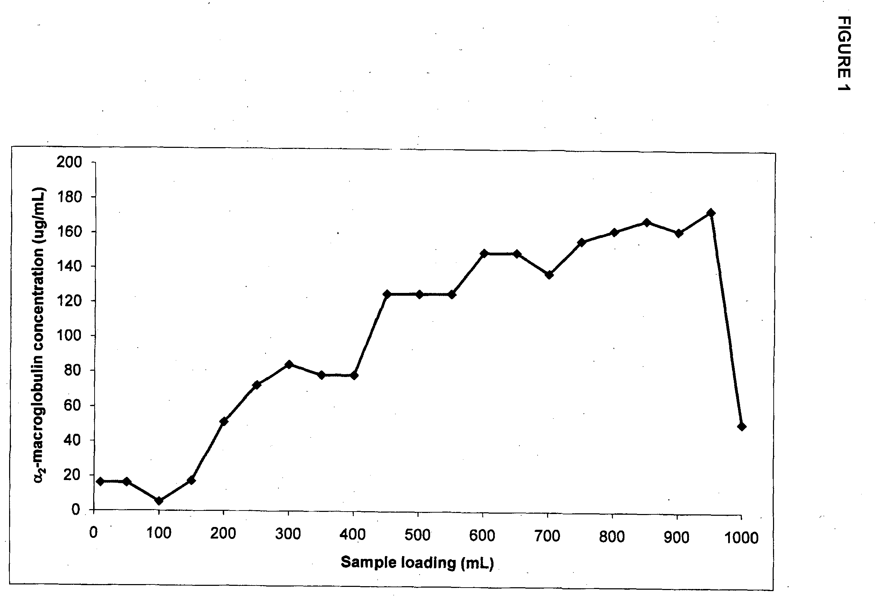

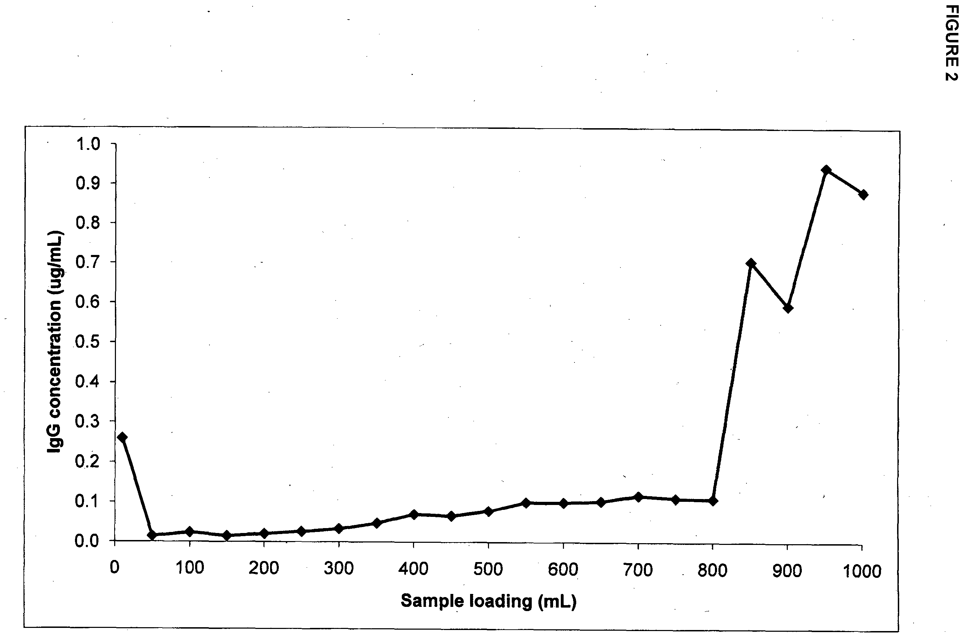

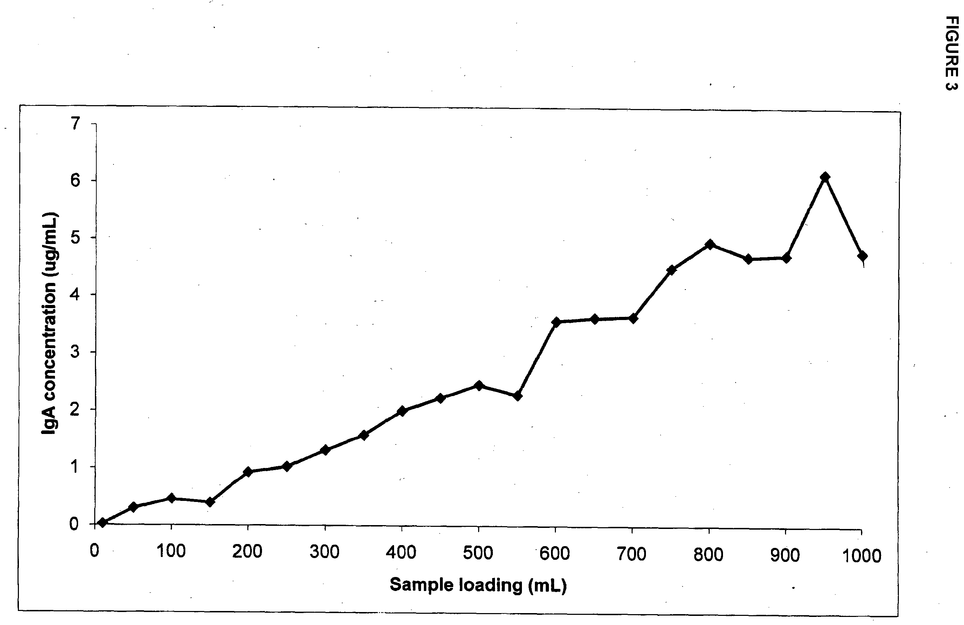

The pH adjusted albumin eluate derived from CM Sepharose-FF (1000 mL) was loaded on to the MEP Hypercel column and the flow through fraction was collected at 10 mL intervals. The impurity break through profiles varied significantly for the four impurity proteins (Figures 1 - 4). During the initial stages of sample loading only minor amounts of IgG were detected in the flow through (Figure 2). Despite a significant increase in IgG break through during the latter stages of the loading, the maximum IgG concentration detected only represented 1.1% of the IgG concentration in the sample load, suggesting that the MEP Hypercel has a high selectivity for IgG. The break through profile for IgA followed an almost linear increase throughout the sample loading (Figure 3). The maximum IgA concentration detected in the flow through represented 9.3% of the IgA concentration in the sample load.

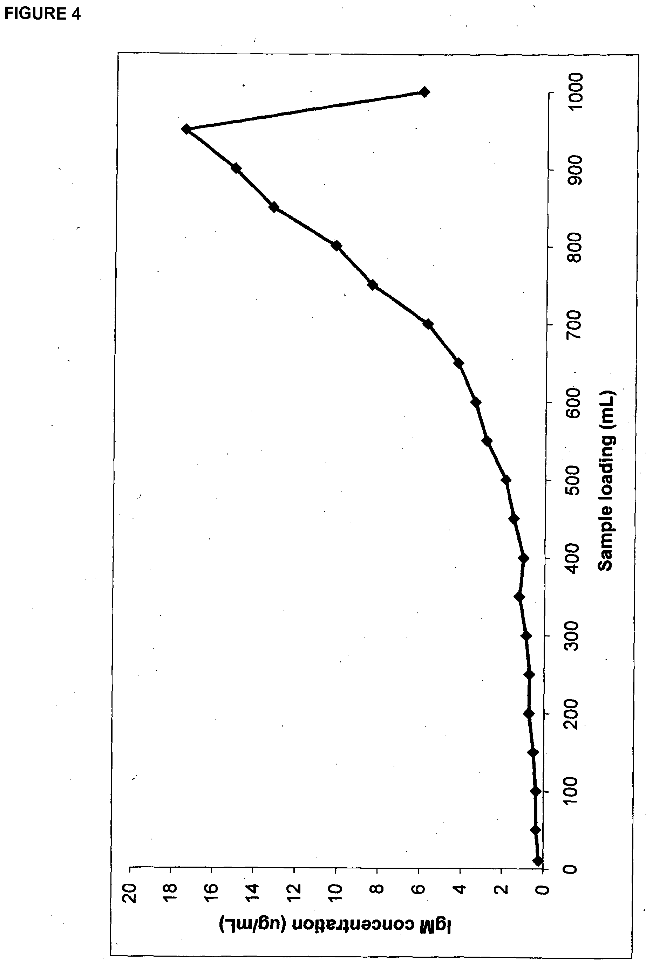

During the initial stages of the sample loading only minor amounts of IgM were detected in the flow through (Figure 4). Towards the latter stages of the sample loading a significant increase in IgM was detected in the flow through. The maximum IgM concentration detected at the later stages of sample loading represented 53.1 % of the IgM concentration of the sample load, suggesting that the capacity or affinity of MEP Hypercel for IgM is significantly lower than for IgG and IgA.

Significant amounts of α₂-macroglobulin were detected in the flow through immediately after commencement of sample loading on to the MEP Hypercel column (Figure 1). The rate of increase in α₂-macroglobulin breakthrough was faster than any of the other impurities and as a result the maximum α₂-macroglobulin concentration detected in the flow through was 76.0% of the α₂-macroglobulin in the sample loading. As the α₂-macroglobulin is the most abundant impurity protein in the albumin eluate derived from CM Sepharose-FF and the MEP Hypercel has the weakest affinity for this protein, it indicates that α₂-macroglobulin will be the key impurity marker for subsequent decisions on the amount of sample that can be loaded on to the MEP Hypercel column.

The initial analysis of the impurity protein break through curves showed that sample loadings of up to 1000 mL on to a 35 mL MEP Hypercel column were excessive. The albumin sample was loaded on to the equilibrated MEP Hypercel column at 100, 200, 300, 400 and 500 mL. The flow through and post-sample wash was collected as a bulk sample along with the pH 3.0 eluate. These bulk fractions were assessed for the levels of albumin by nephelometry and the major impurity proteins of IgG, IgA, IgM and α₂-macroglobulin by ELISA.

The loading of pH adjusted albumin eluate derived from CM Sepharose-FF on to the MEP Hypercel column was explored at sample volumes between 100 - 500 mL. The protein recovery data for the four impurity proteins showed similar trends to the impurity break through profiles previously shown in Figures 1 - 4. The IgG recovery in the flow through fraction was less than 1 % of the total IgG loaded at the highest sample loading (Table 5). At the highest sample loadings the recovery of IgA and IgM was also relatively low at 1.8% and 3.6%, respectively. However, even at the lowest sample loading of 100 mL, 5.2% of the total α₂-macroglobulin loaded on to the MEP Hypercel was recovered in the flow through fraction. This increased proportionally with sample loading, with 35.4% of the total α₂-macroglobulin being recovered in the flow through for the highest sample loading. As α₂-macroglobulin is the predominant impurity protein in the sample load and the MEP Hypercel exhibits the weakest affinity for this impurity protein of all the major impurities, efforts were made to optimise the α2-macroglobulin clearance of MEP Hypercel.

**Table 5: Percentage recovery of proteins in flow through fraction derived from MEP Hypercel chromatography conducted using sample loadings ranging from 100 to 500 mL**

| **Fraction** | **Component** | **Sample loading volume** | | | | |
|---|---|---|---|---|---|---|
| | | **100 mL** | **200 mL** | **300 mL** | **400 mL** | **500 mL** |
| **Flow through** | IgG | 0.01 | 0.03 | 0.04 | 0.05 | 0.06 |
| | IgA | 0.3 | 0.5 | 1.2 | 1.6 | 1.8 |
| | IgM | 1.2 | 1.5 | 1.8 | 2.6 | 3.6 |
| | Albumin | 84.9 | 71.7 | 99.6 | 97.7 | 98.9 |
| | α₂-macroglobulin | 5.2 | 8.8 | 19.8 | 22.2 | 35.4 |

In attempt to improve the selectivity of the MEP Hypercel for all of impurity proteins, the above experiment was repeated with 50 mM NaCl added to the equilibration buffer and albumin sample. The albumin sample was loaded on to the equilibrated MEP Hypercel column at 100, 200, 300, 400 and 500 mL. The flow through and post-sample wash was collected as a bulk sample along with the pH 3.0 eluate. These bulk fractions were assessed for the levels of albumin by nephelometry and the major impurity proteins of IgG, IgA, IgM and α₂-macroglobulin by ELISA.

Earlier data (Table 3) showed that addition of NaCl improved the separation characteristics of MEP Hypercel for pure albumin and IgG solutions. Therefore the impact of 50 mM sodium chloride addition to the pH adjusted albumin sample and equilibration buffer on the removal of impurities from albumin was assessed. The recovery of IgG, IgA, IgM and α₂-macroglobulin in the flow through fractions was almost identical for the MEP Hypercel separations conducted with (Table 5) and without addition of 50 mM sodium chloride (Table 6) indicating that the addition of sodium chloride did not have any impact on the selectivity of MEP Hypercel for the impurity proteins in albumin.

**Table 6: Percentage recovery of proteins in flow through fraction derived from MEP Hypercel chromatography conducted using sample loadings ranging from 100 to 500 mL after addition of 50 mM NaCl to the sample and equilibration buffer**

| | | **Sample loading volume** | | | | |
|---|---|---|---|---|---|---|
| **Fraction** | **Component** | **100 mL** | **200 mL** | **300 mL** | **400 mL** | **500 mL** |
| **Flow through** | IgG | 0.01 | 0.03 | 0.05 | 0.09 | 0.1 |
| | IgA | 0.3 | 1.2 | 2.0 | 1.6 | 1.7 |
| | IgM | 1.7 | 1.6 | 2.2 | 2.9 | 4.3 |
| | Albumin | 99.2 | 103.3 | 113.1 | 106.8 | 119.7 |
| | α₂-macroglobulin | 1.6 | 8.3 | 12.8 | 21.0 | 27.7 |

### Examples 5: Assessment of flow rate on performance of MEP Hypercel

As the four major impurity proteins appeared to have differing affinities to the MEP Hypercel resin, studies were conducted to investigate the impact of the flow rate on the binding of the impurity proteins. Using the chromatography conditions from above, the albumin sample (100 mL) was loaded on to the MEP Hypercel column at flow rates of 50, 100 and 150 cm/h. The flow through and eluate fractions were collected as bulk samples and assessed for the levels of albumin by nephelometry and the major impurity proteins of IgG, IgA, IgM and α₂-macroglobulin by ELISA.

The impact of flow rate on the separation of impurity proteins by MEP Hypercel was assessed between 50 and 150 cm/h. At 50 cm/h less than 1% of each impurity protein was recovered in the flow through fraction (Table 7). Increasing the flow rate caused increased break through of all impurities, but this was more pronounced for IgA and α₂-macroglobulin.

**Table 7: Percentage recovery of proteins in flow through fraction derived from MEP Hypercel conducted at flow rates ranging from 50 to 150 cm/h**

| | | **Flow rate (cm/h)** | | |
|---|---|---|---|---|
| **Fraction** | **Component** | **50** | **100** | **150** |
| **Flow through** | IgG | 0.01 | 0.02 | 0.04 |
| | IgA | 0.8 | 2.4 | 12.4 |
| | IgM | 0.1 | 2.3 | 1.6 |
| | Albumin | 91.3 | 95.8 | 96.2 |
| | α₂-macroglobulin | 0 | 5.3 | 11.3 |

### Example 6 : Impact of upstream process changes

In addition to investigating alternatives for the albumin polishing step, methods of improving the efficiency of the initial anion exchange chromatography step in the chromatographic purification of human serum albumin were assessed. Capto DEAE resin can be used as an alternative to the DEAE Sepharose-FF, and because of its higher capacity, it enables protein loadings to be increased by approximately 50%. Despite the separation characteristics of the DEAE Sepharose-FF and Capto DEAE being similar, some differences were observed. The albumin eluate from the CM Sepharose-FF, which was initially derived from DEAE Sepharose-FF, and the Capto DEAE flow through fraction were initially characterised by nephelometry and ELISA analysis. The two albumin samples were adjusted to pH 7.0 ± 0.1 and clarified by passage through a 0.22 µm membrane (Durapore). The samples were loaded on to a MEP Hypercel column, which had been equilibrated with 110 mM Sodium acetate (pH 7.0), at sample volumes of 100, 200 and 300 mL. The flow through and post-sample wash was collected as a bulk fraction along with the pH 3.0 eluate. The bulk fractions were assessed for levels of albumin by nephelometry and the major impurity proteins of IgG, IgA, IgM and α₂-macroglobulin by ELISA.

The studies into identifying an alternative polishing step for the chromatographic albumin process were conducted in parallel with studies into optimising the efficiency of the albumin capture step. It has been identified that substituting the current DEAE Sepharose-FF with Capto DEAE enables a 50% increase in sample loading [Gruvegard & Allmér, 2009]. The impact of this process change on the MEP Hypercel was assessed. The use of Capto DEAE for initial capture step had some impact on the resultant albumin eluate derived from the CM Sepharose-FF column. The albumin derived from the Capto DEAE process had a lower total impurity content (1.58%) compared to the albumin derived from the DEAE Sepharose-FF process (1.91%) (Table 8). Significant differences were observed in the IgA and α₂-macroglobulin content of the two samples. The albumin derived from the Capto DEAE process had two-fold higher levels of IgA compared to the albumin derived from the DEAE Sepharose-FF process and two-fold lower levels of α₂-macroglobulin.

**Table 8: Impurity analysis of ion exchange purified albumin derived from Capto DEAE and DEAE Sepharose-FF processes**

| | **Albumin from DEAE Sepharose-FF process** | | **Albumin from Capto DEAE process** | |
|---|---|---|---|---|
| **Component** | **Concentration (mg/mL)** | **% of total** | **Concentration (mg/mL)** | **% of total** |
| Albumin | 18.40 | 98.09 | 26.80 | 98.42 |
| IgG | 0.091 | 0.49 | 0.082 | 0.30 |
| IgA | 0.065 | 0.35 | 0.192 | 0.71 |
| IgM | 0.014 | 0.07 | 0.023 | 0.08 |
| α₂-macroglobulin | 0.189 | 1.01 | 0.133 | 0.49 |
| Total impurities | 0.359 | 1.91 | 0.430 | 1.58 |

Following MEP Hypercel chromatography of the ion exchange purified albumin, significant differences were observed between the albumin derived from the Capto DEAE process and the DEAE Sepharose-FF process. The total impurity content of the albumin derived from the DEAE Sepharose-FF process was 0.0637% for the 100 mL MEP Hypercel sample load and increased to 0.2390% for the 300 mL loading (Table 9). The major impurity for these samples was α₂-macroglobulin, which comprised over 80% of the total impurities. The MEP purified albumin derived from the Capto DEAE process had undetectable levels α₂-macroglobulin for all sample loadings, resulting in significantly lower total impurity contents compared to the albumin derived from the DEAE Sepharose-FF process. The results suggest that the significantly lower α₂-macroglobulin levels in the ion exchange purified albumin derived from the Capto DEAE process had a beneficial impact on the MEP Hypercel chromatography, thus permitting higher sample loadings.

**Table 9: Impurity content of MEP Hypercel purified albumin derived from Capto DEAE and Sepharose DEAE chromatography (ND = Not detectable)**

| | **Albumin from DEAE Sepharose-FF process** | | | **Albumin from Capto DEAE process** | | |
|---|---|---|---|---|---|---|
| **Component** | **100 mL loading** | **200 mL loading** | **300 mL loading** | **100 mL loading** | **200 mL loading** | **300 mL loading** |
| Albumin | 99.936 | 99.880 | 99.761 | 99.998 | 99.995 | 99.995 |
| IgG | 0.0001 | 0.0002 | 0.0003 | 0.0001 | 0.0003 | 0.0004 |
| IgA | 0.0072 | 0.0139 | 0.0263 | 0.0010 | 0.0023 | 0.0022 |
| IgM | 0.0019 | 0.0025 | 0.0021 | 0.0011 | 0.0020 | 0.0027 |
| α₂-macroglobulin | 0.055 | 0.10 | 0.21 | ND | ND | ND |
| Total impurities | 0.0637 | 0.1201 | 0.2390 | 0.0023 | 0.0047 | 0.0053 |

### Example 7 : Estimation of process efficiency

To compare the process efficiency of the MEP Hypercel to the existing Sephacryl S200HR, the conditions used for the MEP Hypercel pilot-scale processing were extrapolated to a full-scale batch. This was compared to actual data for the Sephacryl S200HR step. Parameters compared included; flow rate, column size, sample loading, buffer usage and process time.

Using the MEP Hypercel conditions determined at laboratory-scale and verified at pilot-scale, these were extrapolated to a full-scale process to calculate various processing parameters. The results were compared to actual data for the processing of a 10 tonne plasma batch through the Sephacryl S200HR column. The small sample loading and low flow rate used for the Sephacryl S200HR means that a significant amount of chromatographic cycles and time is required to process a 10 t batch (Table 10). This is despite the fact that Sephacryl S200HR column is over 1000 L and the sample is concentrated to approximately 130-135 mg/mL to increase the process efficiency.

The MEP Hypercel column can be loaded with significantly higher amounts of sample and can be operated at flow rates over three times faster than the Sephacryl S200HR (Table 10). This means that a 198 L MEP Hypercel column can process the albumin in approximately half the time required than the Sephacryl S200HR. If an MEP Hypercel column with a similar size as the Sephacryl S200HR is used (5 individual 17.5 x 120 cm ID columns), the 10 ton batch of albumin can be processed in 558 min, which represents an 88% saving compared to the actual processing time required for the Sephacryl S200HR.

The amount of buffer required for the MEP Hypercel chromatography is marginally higher than the Sephacryl S200HR largely as a result of the MEP Hypercel requiring cleaning and regeneration after each cycle compared to the Sephacryl S200HR, which only requires cleaning and regeneration to be conducted at the end of a batch.

A further benefit of the MEP Hypercel is the streamlining of the overall purification process. Currently, the ion exchange eluate requires concentrating from approximately 25 mg/mL to approximately 135 mg/mL via ultrafiltration in order to maximise the efficiency of the Sephacryl S200HR chromatography step. However, this ultrafiltration step is not required prior to MEP Hypercel chromatography. The ion exchange eluate only requires pH adjustment to 7.0 prior to loading on to the MEP Hypercel column.

**Table 10: Comparison of efficiency of polishing chromatographically purified albumin using Sephacryl S200HR (90 cm x 120 cm internal diameter; ID) and MEP Hypercel (17.5 cm x 120 cm ID or 87. 5 cm x 120 cm ID) for a 10 tonne batch of albumin**

| | **Resin type (dimensions)** | | |
|---|---|---|---|
| **Parameter** | **Sephacryl S200HR** | **MEP Hypercel** | **MEP Hypercel** |
| Column volume (L) | 1018 | 198 | 990 |
| Sample load per cycle (L) | 61.1 | 1130 | 5650 |
| Protein concentration of sample (mg/mL) | 130-135 | 20-25 | 20-25 |
| Number of cycle required to process 10 t batch | 40 | 13 | 3 |
| Total volume of product processed for 10 t batch (L) | 2441 | 14449 | 14449 |
| Flow rate (L/min) | 5.6 | 18.9 | 94.3 |
| Buffer consumption per 10 t batch (L) | 26208 | 30872 | 35622 |
| Time required for processing a 10 t batch (min) | 4680 | 2417 | 558 |

### Example 8 : Characterization of albumin fractionated chromatographically from delipidated and euglobulin depleted Cohn Supernatant I incorporating Capto DEAE chromatography (in positive mode with respect to albumin)

Delipidated and euglobulin depleted Cohn Supernatant I (15 kg) was used as the starting material. The solution was clarified using a 0.22 µm filter. A glass column (1374mL) filled with Capto DEAE resin was equilibrated with 10 mM Sodium acetate (pH 5.2). The Delip/Eug SNI was loaded at a protein loading of 100 g/L and a flow rate of 100 cm/h. At the end of sample loading, the column was washed with 3 column volumes of 10 mM Sodium acetate (pH 5.2). The unbound protein collected during sample application and post sample wash was diverted to immunoglobulin processing. The bound protein was selectively eluted. The first elution, which contains albumin, occurs by washing with 3 column volumes of 25 mM Sodium acetate (pH 4.5). The remainder of the bound protein was eluted by washing with 2 column volumes of 1 M NaCl and 2 column volumes of 1 M NaOH.

A glass column (1374 mL) filled with CM Sepharose FF resin was equilibrated with 25 mM Sodium acetate (pH 4.5). The entire albumin eluate from the Capto DEAE was loaded on to the CM Sepharose FF column at a flow rate of 100 cm/h. At the end of sample loading, the column was washed with 2 column volumes of 25 mM Sodium acetate (pH 4.5). The bound protein was selectively eluted. The first elution, which contains albumin, occurs by washing with 3 column volumes of 110 mM Sodium acetate (pH 5.5). The remainder of the bound protein was eluted by washing with 2 column volumes of 1 M NaCl and 2 column volumes of 1 M NaOH. The albumin eluate from CM Sepharose FF was adjusted to pH 7.0 using 1 M NaOH and 1 M Acetic acid.

A glass column (1343 mL) filled with MEP Hypercel resin was equilibrated with 110 mM Sodium acetate (pH 7.0). The pH adjusted CM eluate was loaded at 571% of a column volume at a flow rate of 100 cm/h. After loading, the column was washed with 2 column volumes of 110 mM sodium acetate (pH 7.0). The protein eluted during the sample loading and post sample wash was retained for further processing. The bound protein was eluted from the MEP Hypercel by washing with 2 column volumes of 100 mM Sodium acetate (pH 3.0) and 2 column volumes of 1 M NaOH. The purified albumin was processed according to the procedure for Albumin 2VI.

The albumin (20% solution) derived from the above process was characterised using the batch release testing and a range of additional tests to assess product purity. The data was compared to control batches, which were manufactured using the current process (Table 11).

The albumin derived from the new process contained lower levels of impurities including IgG (< 0.009 mg/mL), apolipoprotein A1 (< 0.0032 mg/mL), IgA (1.7 µg/mL) and α₁-proteinase inhibitor (0.039 mg/mL) compared to the albumin control (Table 11). The lower impurity content of the albumin derived from the new process also resulted in lower levels of aggregates (0.6%) and turbidity levels (2.0 NTU).

**Table 11: Characterisation albumin solution recovered by this method**

| **Test type (Test number)** | | **Laboratory-scale batch number** | |
|---|---|---|---|
| | **Limits / Expected values** | **Test batches (n = 3)** | **Control batches (n = 3)** |
| Acidity or Alkalinity (QB5931) | 6.9 to 7.3 | 7.2 | 7.2 |
| Protein composition - SEHPLC (QB6618) | Aggregates ≤ 10% | 0.6 | 1.7 |
| | Dimer - For information | 1.4 | 1.3 |
| | Monomer - For information | 98.0 | 97.0 |
| | Fragments - For information | 0 | 0 |
| Sodium (QB6365) | 48 to 100 mmol/L | 85.4 | 79.7 |
| Protein composition (QB8222) | ≥ 95% albumin | 100 | 100 |
| Protein concentration (QB3950) | 19.0 to 21.0% w/v | 22.3 | 19.8 |
| Prekallikrein activator (PKA) (QB8270) | 28.6 IU/mL | < 1 | < 1 |
| Aluminium (QA6430) | ≤ 80 µg/L | 104.7 | 26.7 |
| Caprylate (QB6491) | 27.2 to 36.8 mmol/L | 33.6 | 31.0 |
| Viable counts (QB2680) | No viables detected | 0 | 0 |
| Appearance (QB5908) | Clear, slightly viscous liquid with colourless yellow, amber or green | Pass | Pass |
| Osmolality (QB6780) | For information | 134 | 131.7 |
| IgA ELISA (QB3344) | For information | 1.69 | 4.1 |
| IgG (QB4420) | For information | < 0.009 | 0.162 |
| Transferrin (QB0436) | For information | < 0.020 | < 0.020 |
| α₂-macroglobulin (QB0432) | For information | < 0.024 | < 0.024 |
| Apolipoprotein A1 (QB0443) | For information | < 0.0032 | 0.039 |
| α₁-glycoprotein (QB0433) | For information | < 0.012 | < 0.012 |
| α₁-proteinase inhibitor (QB0434) | For information | 0.039 | 0.079 |
| Haptoglobin (QB0438) | For information | < 0.034 | < 0.034 |
| Inter-α-trypsin inhibitor (QB0435) | For information | < 0.063 | < 0.063 |
| Turbidity (QB0496.) | For information | 2.0 | 5.6 |
| Endotoxin (QB9881) | For information | 0.61 | <0.06 |

### Example 9: Characterization of albumin fractionated by CM Sepharose chromatography

The albumin eluate derived from Capto DEAE or DEAE Sepharose FF chromatography was adjusted to pH 5.4 and a conductivity of 5.0 mS/cm using 1 M NaOH and 1 M Acetic acid.

A glass column filled with CM Sepharose FF resin was equilibrated with Sodium acetate (pH 5.4, conductivity 5.0 mS/cm). The adjusted DEAE eluate sample was loaded at a protein loading of 500 g/L and a flow rate of 100 cm/h. At the end of loading, the column was washed with 2 column volumes of Sodium acetate (pH 5.4, conductivity 5.0 mS/cm). The unbound protein collected during sample application and post sample wash was retained for further processing. The bound protein was eluted by washing with 2 column volumes of 1 M NaCl and 2 column volumes of 1 M NaOH. The albumin sample was adjusted to pH 7.0 using 1 M NaOH and 1 M Acetic acid. A glass column filled with MEP Hypercel resin was equilibrated with Sodium acetate (pH 7.0, conductivity 5.0 mS/cm). The pH adjusted albumin was loaded at a protein loading of 150 g/L and a flow rate of 75 cm/h. After loading, the column was washed with 2 column volumes of sodium acetate (pH 7.0, conductivity 5.0 mS/cm). The protein eluted during sample loading and post-sample wash was retained for further processing. The bound protein was eluted from the MEP Hypercel by washing with 2 column volumes of 100 mM Sodium acetate (pH 3.0).

The purity of an albumin sample derived from DEAE Sepharose FF chromatography was characterised after CM Sepharose FF chromatography using the current procedure and the optimised procedure (outlined above). The results show that the current CM Sepharose FF method results in modest increases in the albumin purity (Table 12). This is mainly associated with removal of transferrin. The optimised CM Sepharose FF chromatography results in significant reductions in the levels of transferrin and α₂-macroglobulin, in addition to some reductions in IgG and IgA. As α₂-macroglobulin is the most difficult impurity to remove using the MEP Hypercel, the optimised conditions for the CM Sepharose FF allow higher loadings on to the MEP Hypercel.

**Table 12: Purity analysis of albumin during CM Sepharose FF chromatography**

| **Component** | **% of total protein** | | |
|---|---|---|---|
| | **DEAE Eluate** | **CM Sepharose Eluate** | **Optimised CM Sepharose eluate** |
| Albumin | 97.36 | 98.07 | 99.38 |
| IgG | 0.49 | 0.48 | 0.24 |
| IgA | 0.25 | 0.26 | 0.16 |
| IgM | 0.05 | 0.04 | 0.05 |
| Transferrin | 0.66 | 0.07 | <0.10 |
| α₂-macroglobulin | 1.20 | 1.08 | 0.10 |

### Example 10 : Characterization of chromatographically fractionated albumin from Cohn Supernatant II+III or Filtrate A incorporating Capto DEAE chromatography

SN II + III or Filtrate A was concentrated by ultrafiltration then diafiltered against 5 volumes of PFW to reduce ethanol and salt levels. The diafiltered SN II + III or Fraction A was adjusted to pH 4.5 and a conductivity of 2.0 mS/cm using 1 M NaOH and 1 M Acetic acid. Aerosil 380 was added at 160 g/kg protein to bind lipoproteins. Diacel was added at 400 g/kg protein to facilitate clarification using a filter press. The filter press may be recovered using 25 mM Sodium acetate (pH 4.5, conductivity 2.0 mS/cm).

A glass column filled with Capto DEAE resin was equilibrated with 25 mM Sodium acetate (pH 4.5, conductivity 2.0 mS/cm). The Delipidated and Diafiltered SN II + III sample was loaded at a protein loading of 1000 g/L and a flow rate of 100 cm/h. At the end of loading, the column is washed with 2 column volumes of 25 mM Sodium acetate (pH 4.5, conductivity 2.0 mS/cm). The unbound protein collected during sample application and post sample wash was retained for further processing. The bound protein was eluted by washing with 2 column volumes of 1 M NaCl and 2 column volumes of 1 M NaOH. The albumin sample was adjusted to pH 5.4 and a conductivity of 5.0 mS/cm using 1 M NaOH and 1 M Acetic acid. The procedure for the subsequent CM Sepharose-FF and MEP Hypercel steps are identical to those outlined in Example 10. The Capto DEAE (negative-mode) purification of SN II + III was compared to Capto DEAE conducted in positive-mode (Tables 13 and 14). The results show that the proteins recovered in the drop through fraction from the negative-mode Capto DEAE (Table 14) are the sum of the proteins recovered in the drop through and eluate fractions from the positive-mode Capto DEAE (Table 13). The effectiveness of the negative-mode Capto DEAE is further supported by the fact that the wash fraction (1 M NaCl) for both the positive and negative-mode Capto DEAE purification of SN II + III had similar total protein recoveries. This wash fraction typically contains proteins including IgA, haptoglobin, ceruloplasmin and α₁-glycoprotein, most of which were not measured in this study.

**Table 13: Recovery of proteins during Capto DEAE (positive-mode) purification of SN II+III (ND = Not detected in fraction)**

| | **% of total loaded** | | |
|---|---|---|---|
| **Protein** | **Drop through** | **Eluate** | **Wash** |
| IgG | 90.4 | ND | 2.2 |
| IgA | 48.0 | 15.9 | 36.5 |
| IgM | ND | ND | ND |
| Albumin | 12.9 | 97.1 | 0.4 |
| Transferrin | 86.6 | ND | ND |
| α₂-macroglobulin | ND | ND | ND |
| Total protein | 20.5 | 78.1 | 7.2 |

**Table 14: Recovery of proteins during Capto DEAE (negative-mode) purification of SN II+III (ND = Not detected in fraction)**

| | **% of total loaded** | |
|---|---|---|
| **Protein** | **Drop through** | **Wash** |
| IgG | 103.7 | ND |
| IgA | 98.7 | 20.0 |
| IgM | ND | ND |
| Albumin | 86.7 | 0.2 |
| Transferrin | 97.8 | ND |
| α₂-macroglobulin | ND | ND |
| Total protein | 102.9 | 6.9 |

Given that the SN II + III sample contains high levels of transferrin and that transferrin co-elutes with albumin during negative-mode Capto DEAE chromatography, the ability of the negative-mode CM Sepharose-FF to remove transferrin from albumin was assessed. The protein recovery for the negative-mode CM Sepharose-FF purification of SN II + III show that high levels of transferrin were recovered in the wash fraction (91.3%) and undetectable levels were recovered in the albumin fraction (Table 15). Overall, the protein recoveries for the SN II + III sample were similar to those observed for negative-mode CM Sepharose-FF of albumin derived from SNI.

**Table 15: Recovery of proteins during CM Sepharose-FF (negative-mode) purification of SN II + III (ND = Not detected in fraction; D# = Detectable levels in fraction, however, not detectable in starting material meaning that protein recovery could not be determined)**

| | **% of total loaded** | |
|---|---|---|
| **Protein** | **Drop through** | **Wash** |
| IgG | 66.8 | 31.4 |
| IgA | 75.9 | 20.8 |
| IgM | ND | ND |
| Albumin | 88.8 | 0.4 |
| Transferrin | ND | 91.3 |
| α₂-macroglobulin | ND | D# |
| Total protein | 81.1 | 9.6 |

### Example 11 : Characterisation of albumin fractionated chromatographically from Delipidated Cohn Supernatant II + III utilising ANX Sepharose, CM Sepharose and MEP Hypercel in negative mode

Delipidated Cohn Supernatant II + III was used as the starting material and adjusted to pH 4.7 and a conductivity of 1.8 mS/cm. The solution was clarified using a 0.22 µm filter. A glass column (35 mL) filled with ANX Sepharose-FF resin was equilibrated with Sodium acetate (pH 4.7, conductivity 1.8 mS/cm). The adjusted Delipidated SN II + III was loaded at a protein loading of 300 g/L and a flow rate of 100 cm/h. At the end of sample loading, the column was washed with 3 column volumes of Sodium acetate (pH 4.7, conductivity 1.8 mS/cm). The unbound protein collected during sample application and post sample wash was collected and retained for albumin processing. The bound protein was eluted by washing with 2 column volumes of 1 M NaCl and 2 column volumes of 1 M NaOH.

A glass column (35 mL) filled with CM Sepharose FF resin was equilibrated with Sodium acetate (pH 5.4, conductivity 5.0 mS/cm). The albumin flow through from the ANX Sepharose-FF was adjusted to pH 5.4 and a conductivity of 5.0 mS/cm and clarified using a 0.22 µm filter. The adjusted ANX flow through was loaded on to the CM Sepharose FF column loading of 300 g/L and at a flow rate of 100 cm/h. At the end of sample loading, the column was washed with 2 column volumes of Sodium acetate (pH 5.4, conductivity 5.0 mS/cm). The unbound protein eluted during sample application and post sample wash was collected and retained for albumin processing and characterisation testing. The bound protein was eluted by washing with 2 column volumes of 1 M NaCl and 2 column volumes of 1 M NaOH.

The albumin flow through from CM Sepharose FF was adjusted to pH 7.0 using 1 M NaOH and 1 M Acetic acid. A glass column (35 mL) filled with MEP Hypercel resin was equilibrated with 110 mM Sodium acetate (pH 7.0). The pH adjusted CM flow through was loaded at 150 g/L and at a flow rate of 75 cm/h. After loading, the column was washed with 2 column volumes of 110 mM sodium acetate (pH 7.0). The unbound protein eluted during the sample loading and post sample wash was retained for characterisation testing. The bound protein was eluted from the MEP Hypercel by washing with 2 column volumes of 100 mM Sodium acetate (pH 3.0) and 2 column volumes of 1 M NaOH.

The albumin solutions derived from the above process after the CM Sepharose-FF and MEP Hypercel process steps were characterised using specific ELISA's to assess product purity. The data was compared to control samples of concentrated CM eluate and post size exclusion concentrated albumin monomer, which were manufactured using CSL Biotherapies current albumin purification process.

The albumin intermediate obtained after loading Delipidated SN II + III on to the ANX Sepharose-FF and CM Sepharose-FF operated in negative mode had a higher purity (99.16%) that the equivalent albumin intermediate derived from the current process (98.41%) (Table 16). This difference was mainly attributed to significantly lower levels of α₂-macroglobulin and α₁-antitrypsin. The albumin derived from the negative mode ANX Sepharose and CM Sepharose did however contain more IgG (0.46%) than the albumin derived from the current process (0.28%).

**Table 16: Protein composition of CM eluate (current process) and albumin derived from negative mode ANX Sepharose FF and CM Sepharose FF chromatography**

| | **% of total protein** | |
|---|---|---|
| **Protein Component** | Post CM Sepharose (from current process) | Albumin derived from negative mode ANX Sepharose-FF and CM Sepharose-FF chromatography |
| **IgG** | 0.28 | 0.46 |
| **IgA** | 0.23 | 0.18 |
| **IgM** | 0.07 | 0.00 |
| **Albumin** | 98.41 | 99.16 |
| **a₁-antitrypsin** | 0.23 | 0.06 |
| **Haptoglobin** | 0.02 | 0.03 |
| **Ceruloplasmin** | 0.00 | 0.00 |
| **α₂-macroglobulin** | 0.74 | 0.10 |
| **Transferrin** | 0.00 | 0.01 |
| **α₁-glycoprotein** | 0.01 | 0.00 |

The albumin derived from MEP Hypercel had a high purity (99.96%) above that observed for albumin derived from the current process (99.90%) (Table 17). The albumin purified using MEP Hypercel contained low IgG levels (0.01 %).

**Table 17: Protein composition of Albumin monomer (derivedfrom Sephacryl S200HR) and albumin derived from negative mode ANX Sepharose-FF, CM Sepharose-FF and MEP Hypercel chromatography.**

| | **% of total protein** | |
|---|---|---|
| **Protein Component** | Albumin monomer (from current process) | Albumin derived from negative mode ANX Sepharose-FF, CM Sepharose-FF and MEP Hypercel chromatography |
| **IgG** | 0.09 | 0.01 |
| **IgA** | 0.01 | 0.01 |
| **α₂-macroglobulin** | < 0.01 | 0.02 |
| **Albumin** | 99.90 | 99.96 |

The data support the use of a chromatographic step (in negative mode with respect to albumin) employing an affinity matrix such as MEP Hypercel for the polishing of albumin derived from different sources (*e.g*., SNI, II+III or Filtrate A) and different conditions for the preceding ion exchange chromatographic steps. The fact that the negative mode ion exchange steps target binding of impurity proteins makes membrane chromatography more attractive and could be transferred to industrial membrane chromatography formats.

The ion-exchange chromatography steps used in existing industrial scale processes (such as those used by CSL, Australia to manufacture Albumex, comprising DEAE Sepharose-FF and CM Sepharose-FF, are very effective for the purification of human serum albumin. The delipidated and euglobulin depleted Cohn SNI intermediate, which is loaded on to the initial DEAE Sepharose-FF column, has a purity of approximately 70% and increases to over 98% after processing through the two ion exchange columns. This ion exchange process selects proteins with pI values between 4.5 and 5.2. Prin *et al.* [1995] showed that the isoelectric point (pI) ranges of IgG, IgA and IgM are broad and overlap the pI window selected for by the DEAE Sepharose-FF and CM Sepharose-FF columns in the Albumex process. Given this broad pI range of the immunoglobulins, ion-exchange chromatography cannot be expected to remove all immunoglobulin contaminants from the albumin and thus highlights the need for an orthogonal mode of albumin purification.

The experiments described herein show that equilibration and sample pH may be important parameter for the separation of albumin and IgG on the MEP Hypercel under certain conditions. The molarity and type of buffer do not appear to have any noticeable impact on separation at pH 7.0. As the albumin is eluted from the CM Sepharose-FF column at pH 5.5 in a 110 mM sodium acetate buffer, the albumin would only require a minor pH adjustment prior to loading on the MEP Hypercel column.

The addition of sodium chloride to the sodium acetate buffer at pH 5.5 improved the selectivity between IgG and albumin. Previous studies have shown that the IgG binding capacity of MEP Hypercel is not enhanced [Schwartz *et al.,* 2001] or can even be reduced [Chen *et al.,* 2008] by the addition of salts. However, these previous studies were conducted using samples at neutral pH values, as compared to a pH 5.5 used in the current study, which is relatively close to the pKa of the MEP ligand at 4.8 [Burton & Harding, 1998]. It was shown that the addition of 50 mM sodium chloride to the sodium acetate buffer at pH 7.0 did not improve the removal of impurity proteins from the albumin derived from CM Sepharose-FF.

Break through analysis during loading of the pH adjusted albumin eluate derived from CM Sepharose-FF onto MEP Hypercel showed significant differences in the binding of the four major impurity proteins that were investigated. The MEP Hypercel showed highest capacity for both IgG and IgA, and much lower capacity for IgM and α₂-macroglobulin. The reduced capacity may be attributed, at least in part, to steric hindrance and size exclusion effects. This is supported further by the fact that α₂- macroglobulin, which has a similar molecular mass to IgM, also had a low binding capacity on the MEP Hypercel. Another possible contributing factor for the low capacity of MEP Hypercel for α₂- macroglobulin is that it has a higher solubility than the gamma globulins (IgG, IgA, and IgM) in ethanol, with the α-globulins being isolated in Cohn Fraction IV [Cohn *et al.,* 1946].

The MEP Hypercel appears to have broader selectivity for the impurities in the chromatographically purified albumin. Not only can the MEP Hypercel remove the high molecular weight impurities, but it can also remove significant amounts of apolipoprotein A1. The Sephacryl S200HR used in many existing methods for the chromatographic purification (polishing) of albumin does not remove any apolipoprotein A1 from albumin as its molecular mass (28 kDa) is smaller than albumin and therefore co-elutes with the albumin monomer fraction.

Incorporation of MEP Hypercel into existing processes such as the Albumex 20™ manufacturing process (CSL, Australia) has very minimal impact on the upstream and downstream processes. In the current Albumex 20 process, for example, the albumin eluted from the Sephacryl S200HR column is in a 50 mM acetate buffer and is diafiltered against water to remove the acetate and sodium ions. The purified albumin derived from the MEP Hypercel column is in a 110 mM sodium acetate buffer and therefore would require an additional diafiltration volume in order to achieve an equivalent product to the existing process. However, the additional time required to conduct the extra diafiltration is not significant. An additional benefit to the significant increase in process efficiency afforded by the MEP Hypercel is that the purified albumin product no longer requires concentrating following elution from the CM Sepharose-FF (ion exchange) column. Omission of this step also results in significant time savings and considerable ongoing cost savings associated with no longer requiring to maintain the ultrafiltration equipment. One of the major attractions to the use of MEP Hypercel as a media for separation based on the hydrophobicity of proteins is its ability to separate without the requirement to add lyotropic salts. Based on the process efficiency calculations for a 10 tonne batch of albumin and assuming that an HIC separation would require an ammonium transfer concentration of 1M in the sample and equilibration buffer, over 3 tonnes of ammonium sulphate would be required. This presents a range of issues including cost, handling, additional downstream processing to remove the salt and disposal, particularly for ammonium transfer as it can cause eutrophication if released into waterways. The efficiency gains afforded by the MEP Hypercel mean that this polishing step is not longer a bottleneck in the process. It also means that the column size can be significantly reduced, which results in a smaller footprint in the manufacturing facility and less resin volumes to be held in inventories.

Demonstrated herein is the effectiveness of affinity chromatography in negative mode (with respect to albumin), in particular, MEP Hypercel chromatography, as a polishing step for ion exchange chromatographically purified albumin by exploiting differences in the hydrophobicity of impurity proteins compared to albumin.

It is anticipated that MEP Hypercel will offer a method of polishing human serum albumin derived from other purification schemes, particularly those that do not precipitate Fraction V. This method will be particularly relevant to the smaller plasma fractionators who already have chromatographic purification processes similar to, for example, the Albumex 20 process. It should also prove appealing to smaller companies and countries that are planning to establish their own plasma fractionation processes, for which chromatographic purification processes are well suited.

The present invention offers significant improvements in process efficiency compared to the existing gel filtration chromatography and can be seamlessly integrated into an existing albumin manufacturing process as the desired separation can be achieved using the sodium acetate buffers that are already used at the preceding steps and does not require the addition of lyotropic salts. This polishing method has immediate benefit for the smaller volume plasma fractionators who employ chromatographic methods, however the significant efficiency gains afforded by the MEP Hypercel could make the chromatographic purification of albumin more attractive for medium to large volume commercial plasma fractionators.

### References

Arakawa T, Kita Y, Sato H, Ejima D (2009) MEP chromatography of antibody and Fc-fusion protein in aqueous arginine solution. Protein Expression and Purification 63:158-163.
Arakawa T, Futatsumori-Sugai M, Tsumoto K, Kita Y, Sato H, Ejima D (2010) MEP Hypercel chromatography II: Binding, washing and elution. Protein Expression and Purification 71:168-173.
Berglof JH, Eriksson S, Curling JM (1983) Chromatographic preparation and in vitro properties of albumin from human plasma. J App Biochem 5:282-292.
Bertolini J, Davies J, Wu J, Pritchard K, Seneviratne G, Stuckley K, Fogarty K; Goss NH (1999) Chromatographic purification of immunoglobulins. Plasma Product Biotechnology Meeting, Daydream Island, Australia, March 27-30.
Burton SC, Harding DRK (1998) Hydrophobic charge induction chromatography: salt independent protein adsorption and facile elution with aqueous buffers. J Chromatogr A 814:71-81.
Cabrera-Crespo J, Goncalves VM, Martins EA, Grellet S, Lopes AP, Raw I (2000) Albumin purification from human placenta. Biotechnol Appl Biochem 31:101-106.
Campbell WR, Hanna MI (1937) The albumin, globulins and fibrinogen of serum and plasma. J Biol Chem 119(1):15-33.
Che Y, Wilson FJ, Bertolini J, Schiff P, Maher DW (2006) Impact of manufacturing improvements on clinical safety of albumin: Australian pharmacovigilance data for 1988-2005. Crit Care Resusc 8(4):334-338.
Chen J, Tetrault J, Ley A (2008) Comparison of standard and new generation hydrophobic interaction chromatography resins in the monoclonal antibody purification process. J Chromatogr A 1177:272-281.
Cohn EJ, Strong LE, Hughes WL, Mulford DJ, Ashworth JN, Melin M, Taylor HL (1946) Preparation and properties of serum and plasma proteins. IV. A system for the separation into fractions of the protein and lipoprotein components of biological tissues and fluids. J Am Chem Soc 68:459-475.
Coulon D, Cabanne C, Fitton V, Noubhani AM, Saint-Christophe E, Santarelli X (2004) Penicillin acylase purification with the aid of hydrophobic charge induction chromatography. Journal of Chromatography B 808:111-115.
Cullen GE, Van Slyke DD (1920) Determination of the fibrin, globulin and albumin nitrogen of blood plasma J Biol Chem 41:587-597.
Ghose S, Hubbard B, Cramer S (2006) Evaluation and comparison of alternatives to Protein A chromatography - Mimetic and hydrophobic charge induction chromatographic stationary phases. Journal of Chromatography A 1122:144-152.
Goheen SC, Matson RS (1985) Purification of human serum gamma globulins by hydrophobic interaction high-performance liquid chromatography. Journal of Chromatography 326:235-241.
Gruvegard M, Allmér K (2009) Designing next generation chromatography media for modern high-throughput plasma processes. Plasma Product Biotechnology Meeting, Menorca, Spain, May 11-15.
Kistler P, Nitschmann HS (1962) Large-scale production of human plasma fractions. Vox Sang 7:414-424.
Marrs SB (1993) Large scale albumin fractionation by chromatography. In Biotechnology if Blood Proteins (Rivat C, Stoltz J-F eds.) Colloque INSERM/John Libbey Eurotext Ltd. pp. 169-173.
Martins EA, Cheng E, Raw I (2011) Plasma fractionation facility at Instituto Butantan - Sao Paulo - Brazil. Plasma Product Biotechnology Meeting, Paphos, Cyprus, May 2011.
Melander W, Horvath C (1977) Salt effects on hydrophobic interactions in precipitation and chromatography of proteins; an interpretation of the lyotropic series. Archives of Biochemistry and Biophysics 183:200-215.
More JE, Harvey MJ (1991) Purification technologies for human plasma albumin. In Blood Separation and Plasma Fractionation (Harris JR ed.) Wiley-Liss, New York, pp. 261-306.
Ohmura T, Sumi A, Ohtani W, Fuluhata N, Takeshima K, Kamide K, Noda M, Kondo M, Ishikawa S, Oohara K, Yokoyama K (1995) Recombinant human serum albumin, process for producing the same and pharmaceutical preparation containing the same. US Patent 5440018.
Pezzini J, Joucla G, Gantier R, Toueille M, Lomenech A-M, Le Senechal C, Garbay B, Santarelli X, Cabanne C (2011) Antibody capture by mixed mode chromatography: A comprehensive study from determination of optimal purification conditions to identification of contaminating host cell proteins. Journal of Chromatography A 1218:8197-8208.
Prin C, Bene MC, Gobert B, Montagne P, Faure GC (1995) Isoelectric restriction of human immunoglobulin isotypes. Biochim Biophys Acta, 1243:287-290.
Ramos-Clamont G, del Carmen Candia-Plata M, Zamudio RG, Vazquez-Moreno L (2006) Novel hydrophobic interaction chromatography matrix for specific isolation and simple elution of immunoglobulins (A, G and M) from porcine serum. Journal of Chromatography A 1122:28-34.
Rucheton M, Stefas E, Graafland H (1997) Method for purifying an aqueous solution of raw albumin. US Patent 5677424.
Schwartz W, Judd D, Wysocki M, Guerrier L, Birck-Wilson E, Boschetti E (2001). Comparison of hydrophobic charge induction chromatography with affinity chromatography on protein A for harvest and purification of antibodies. J Chromatogr A, 908:251-263.
Véron J-L, Gattel P, Pla J, Fournier P, Grandgeorge M (1993) Combined Cohn/chromatography purification process for the manufacture of high purity human albumin from plasma. In Biotechnology if Blood Proteins (Rivat C, Stoltz J-F eds.) Colloque INSERM/John Libbey Eurotext Ltd. pp. 183-188.
Yap HB, Young IF, Micucci V, Herrington RW, Turner PJ, Davies JR (1993) Development of a process for the preparation of human serum albumin using chromatographic methods. In Biotechnology if Blood Proteins (Rivat C, Stoltz J-F eds.) Colloque INSERM/John Libbey Eurotext Ltd. pp. 143-149.

## Claims

1. A method of polishing albumin to remove contaminants comprising passing an albumin enriched solution through a hydrophobic charge-induction chromatographic resin and recovering the albumin solution which passes through the resin.

2. The method of claim 1, wherein the hydrophobic charge-induction chromatographic resin comprises 4-mercaptoethylpyridine.

3. The method of claim 1 or 2, wherein the albumin enriched solution is selected from Cohn Supernatant I, Cohn Supernatant II+III, Supernatant/Filtrate A, Cohn Supernatant IV and Fraction V.

4. The method of any one of claims 1 to 3, wherein the albumin enriched solution is a diafiltered Cohn Supernatant I.

5. The method of any one of claims 1 to 4, wherein the albumin enriched solution is delipidated and euglobulin depleted.

6. The method of any one of claims 1 to 5 wherein, prior to passing the albumin enriched solution through the hydrophobic charge-induction chromatographic resin, the method comprises the steps of (a) passing the solution through an anion-exchange resin in negative mode with respect to albumin and (b) passing the solution through a cation exchange resin in negative mode with respect to albumin.

7. The method of claim 6 comprising passing the flow through fraction from the anion-exchange resin of step (a) through the cation-exchange resin in step (b).

8. The method of claim 6 comprising passing the flow through fraction from the cation-exchange resin of step (b) through the anion-exchange resin in step (a).

9. The method of any one of claims 1 to 8 comprising pasteurizing the polished albumin solution.

## Patentansprüche

1. Verfahren zum Feinreinigen von Albumin zur Entfernung von Verunreinigungen, umfassend das Leiten einer albuminangereicherten Lösung durch ein Harz für die hydrophobe Ladungsinduktionschromatographie und das Gewinnen der Albuminlösung, die durch das Harz hindurchgeht.

2. Verfahren gemäß Anspruch 1, wobei das Harz für die hydrophobe Ladungsinduktionschromatographie 4-Mercaptoethylpyridin umfasst.

3. Verfahren gemäß Anspruch 1 oder 2, wobei die albuminangereicherte Lösung aus Cohn Supernatant I, Cohn Supernatant II+III, Supernatant/Filtrate A, Cohn Supernatant IV und Fraction V ausgewählt wird.

4. Verfahren gemäß einem der Ansprüche 1 bis 3, wobei die albuminangereicherte Lösung ein diafiltriertes Cohn Supernatant I ist.

5. Verfahren gemäß einem der Ansprüche 1 bis 4, wobei die albuminangereicherte Lösung delipidiert und euglobulinabgereichert ist.

6. Verfahren gemäß einem der Ansprüche 1 bis 5, wobei das Verfahren vor dem Leiten der albuminangereicherten Lösung durch das Harz für die hydrophobe Ladungsinduktionschromatographie die Schritte (a) Leiten der Lösung durch ein Anionenaustauscherharz im Negativmodus hinsichtlich Albumin und (b) Leiten der Lösung durch ein Kationenaustauscherharz im Negativmodus hinsichtlich Albumin umfasst.

7. Verfahren gemäß Anspruch 6, umfassend das Leiten der Durchflussfraktion von dem Anionenaustauscherharz von Schritt (a) durch das Kationenaustauscherharz in Schritt (b).

8. Verfahren gemäß Anspruch 6, umfassend das Leiten der Durchflussfraktion von dem Kationenaustauscherharz von Schritt (b) durch das Anionenaustauscherharz in Schritt (a).

9. Verfahren gemäß einem der Ansprüche 1 bis 8, umfassend das Pasteurisieren der feingereinigten Albuminlösung.

## Revendications

1. Procédé de polissage d'albumine pour enlever des contaminants comprenant le passage d'une solution enrichie en albumine dans une résine chromatographique d'interaction hydrophobe à induction de charge et la récupération de la solution d'albumine qui passe dans la résine.

2. Procédé selon la revendication 1, dans lequel la résine chromatographique d'interaction hydrophobe à induction de charge comprend de la 4-mercaptoéthylpyridine.

3. Procédé selon la revendication 1 ou 2, dans lequel la solution enrichie en albumine est choisie entre du surnageant de Cohn I, du surnageant de Cohn II+III, du surnageant/filtrat A, du surnageant de Cohn IV et de la fraction V.

4. Procédé selon l'une quelconque des revendications 1 à 3, dans lequel la solution enrichie en albumine est un surnageant de Cohn I diafiltré.

5. Procédé selon l'une quelconque des revendications 1 à 4, dans lequel la solution enrichie en albumine est délipidée et appauvrie en euglobulines.

6. Procédé selon l'une quelconque des revendications 1 à 5, le procédé, avant le passage de la solution enrichie en albumine dans la résine chromatographique d'interaction hydrophobe à induction de charge, comprenant les étapes de (a) passage de la solution dans une résine échangeuse d'anions en mode négatif par rapport à l'albumine et (b) passage de la solution dans une résine échangeuse de cations en mode négatif par rapport à l'albumine.

7. Procédé selon la revendication 6 comprenant le passage de la fraction sortant de la résine échangeuse d'anions de l'étape (a) dans la résine échangeuse de cations dans l'étape (b).

8. Procédé selon la revendication 6 comprenant le passage de la fraction sortant de la résine échangeuse de cations de l'étape (b) dans la résine échangeuse d'anions dans l'étape (a).

9. Procédé selon l'une quelconque des revendications 1 à 8 comprenant la pasteurisation de la solution d'albumine ayant subi un polissage.
